# EUROPÄISCHE PATENTSCHRIFT

(11) **EP 3 562 932 B1**
(45) Veröffentlichungstag und Bekanntmachung des Hinweises auf die Patenterteilung: **13.05.2020**
(21) Anmeldenummer: 17828934.4
(22) Anmeldetag: 28.12.2017
(51) Int. Cl.: C12M 1/22, C12M 1/00, C12M 1/26

(54) **PERFUSIONSVORRICHTUNG**
PERFUSION DEVICE
DISPOSITIF DE PERFUSION

(30) Priorität: 30.12.2016 AT 511952016
(43) Veröffentlichungstag der Anmeldung: 06.11.2019
(73) Patentinhaber: Hektros S.R.L, 39100 Bozen (IT)
(72) Erfinder: HEKL, Daniel, 6020 Innsbruck (AT); TROGER, Hermann, 39042 Brixen (IT)
(74) Vertreter: Torggler & Hofinger Patentanwälte
(86) Internationale Anmeldenummer: PCT/EP2017/084732
(87) Internationale Veröffentlichungsnummer: WO 2018/122322

(56) Entgegenhaltungen:
- WO-A1-2004/094060
- WO-A1-2013/144253
- DE-A1- 10 118 905

## Beschreibung

Die Erfindung betrifft eine Perfusionsvorrichtung für ein Zellkulturbehältnis.

Eine Methode das Verhalten von Zellen oder Mikroorganismen zu untersuchen, besteht darin, Kulturen in Zellkulturbehältnissen (sogenannte Kulturschalen) zu züchten und dann die Auswirkungen eines gegebenenfalls mit Medikamenten oder anderen Testsubstanzen versetztes Nährmedium zu beobachten. Bei den Zellkulturbehältnissen handelt es sich um schalenförmige Behältnisse wie etwa Petrischalen, um zumindest teilweise plattenförmige Behältnisse wie zum Beispiel Multiwellplatten oder auch andere Behältnisse, die zur Kultivierung von Zellen oder Mikroorganismen verwendet werden.

Nachdem die Zellen in das Zellkulturbehältnis eingesät sind, beginnen die Zellen sich zu vermehren bis die gesamte Wachstumsoberfläche des Behältnisses mit Zellen bewachsen ist. Dabei werden die Zellen mit dem Behältnis im Fall einer statischen Kultur (sogenanntes "batch-Verfahren") in einen speziellen Brutschrank ruhend gestellt, der bei Körpertemperatur eine wasserdampfgesättigte Atmosphäre aus 95% Luft und 5% Kohlendioxid konstant hält. Sauerstoff erhalten die mit dem Nährmedium statisch überschichteten Zellen somit durch passive Diffusion über einen Spalt, des nicht dicht aufliegenden Deckels des Behältnisses. Hauptproblem sind dabei die sich über die gesamte Kulturdauer kontinuierlich verändernden Umgebungsbedingungen in statischen Kulturen. Nährstoffe werden von den Zellen aus dem frisch überschichteten Nährmedium aufgenommen und in chemisch abgewandelter Form "verstoffwechselt" wieder ins Nährmedium abgegeben. Dabei benötigen die stoffwechselaktiven Zellen in den ersten 12 bis 24 Stunden des Nährstoffüberangebots sehr viel Sauerstoff zum oxidativen Abbau von Glukose und Aminosäuren, wobei sich rasch ein diffusionsbedingten Sauerstoffgradienten im millimeterdick überschichteten Nährmedium ausbildet. Die Zellen werden häufig hypoxisch und passen ihren Stoffwechsel vermehrt auf anaerobe Glykolyse an. Dabei wird Glukose nicht mehr zu CO₂, sondern fast nur noch zu Laktat abgebaut und ausgeschieden. Üblicherweise sind die im begrenzten Volumen des überschichteten Nährmediums enthaltenen Nährstoffe nach 24 bis 48 Stunden Kulturdauer komplett verbraucht, während sich das Nährmedium sich gleichzeitig mit Ausscheidungsprodukten des Zellstoffwechsels anreichert. Aufgrund des damit verbundenen Unterangebots an Nährstoffen haben die Zellen ihren Stoffwechsel heruntergefahren, wobei das anfänglich leicht basische Nährmedium in der Regel aufgrund der Laktatausscheidung leicht sauer und leicht toxisch wird, zum Beispiel in Folge einer Anreicherung mit Ammoniak aus dem Abbau von Glutamin.

Zusätzlich werden die Zellen bei der anaeroben Glykolyse energetisch geschwächt und der Sauerstoffgehalt im Medium kehrt wieder in den Sättigungsbereich zurück. Dabei kann plötzlich sogar ein Überangebot an Sauerstoff entstehen, der mangels Nährstoffe auch nicht mehr veratmet bzw. benötigt wird. Das schädigt die Zellen wegen vermehrter Abwehrschwäche durch kaum noch vorhandene Radikalfänger-Moleküle gegenüber sich ständig neu bildender Sauerstoffradikale. Ist das Nährmedium verbraucht, wird es abgesaugt und die Zellen mit neuerlich frischem Nährmedium überschichtet. Der Überfluss-Hunger-Zyklus beginnt von vorne.

In statischen Kulturen können aufgrund dieser zyklischen Kulturinhomogenität kaum verlässlich reproduzierbare Versuche mit gleichförmiger Aussagekraft durchgeführt werden. Beispielsweise ist für die Gabe von Medikamenten der Zeitpunkt von extremer Bedeutung. Werden die Medikamente zu Beginn des Fütterungszyklus zugeführt, sind die Zellen in einem guten energetischen Zustand und sehr stoffwechselaktiv. Sie reagieren mitunter völlig anders, sobald sich der Stoffwechsel auf den nachfolgend immer schlimmer werdenden Hungerzustand umstellt. Zusammengefasst kann festgestellt werden, dass gezüchtete Zellen im klassischen Batch-Verfahren zu keinem Zeitpunkt der Kulturdauer homogene Kulturbedingungen vorfinden. Dementsprechend schwierig sind aussagekräftige Versuchsergebnisse zu Medikamentenwirkungen in vitro zu erzielen. Dennoch werden statische Kulturen häufig für Untersuchungen herangezogen, da es sich um ein einfaches System mit niedrigen Kosten und hohem Probendurchsatz handelt.

Zur Behebung der Nachteile statischer Verfahren sind im Stand der Technik sogenannte Perfusionskulturverfahren bekannt geworden, welche mehrere wissenschaftlich belegte Vorteile gegenüber der klassischen Methode zur Kultivierung von Zellen aufweisen. Beispielsweise wird ähnlich wie im Körper dafür gesorgt, dass die gezüchteten Zellen über die gesamte Kulturdauer gleichförmigen Umgebungsbedingungen ausgesetzt sind. Herkömmliche Perfusionsvorrichtungen weisen spezielle, leicht überdruckdichte Perfusionskammern auf, in denen die Zellen kultiviert werden. Über ein Schlauchsystem und eine Pumpe wird Nährmedium aus einem Nährmedien-Reservoir der Perfusionskammer zugeführt. Das Reservoir wird meist auf Körpertemperatur gebracht und mit einem Luft-CO₂-Gemisch analog zum Brutschrank begast. Auf diese Weise wird dafür Sorge getragen, dass die Zellen bei einer regulierbaren Flussrate durch die Perfusionskammer kontinuierlich frisches Medium nach Bedarf zugeführt bekommen. Es herrschen somit konstante Umgebungsbedingungen über die gesamte Kulturdauer. Das wiederum gewährleistet aussagekräftige und reproduzierbare Versuchsergebnisse, die weitestgehend unabhängig vom Verabreichungszeitpunkt von Testsubstanzen erzielt werden können.

Die US 8,501,462 zeigt eine Perfusionsvorrichtung mit einer Multiwellplatte, bei der mehrere Einsätze vorgesehen sind, die jeweils über einen Perfusionsbereich verfügen. An der Unterseite der Einsätze ist eine gasdurchlässige Membran angeordnet, wobei jede Membran, an der dem jeweiligen Einsatz zugewandten Seite Vorsprünge aufweist, mittels der die Membran von der Unterseite des Einsatzes beabstandet wird. Der Zwischenraum zwischen den Vorsprüngen bildet einen Flusskanal, durch den das Nährmedium strömen kann und dabei über die Membran zu den Zellkulturen diffundiert. Nachteilig bei dieser Ausgestaltung ist, dass das Nährmedium zum Großteil auf dem direkten Weg von der Zuleitung zur Ableitung fließt. Auch an den polygonalen Rändern der Einsätze kommt es zu einer unterschiedlichen Nährstoffverfügbarkeit, aufgrund einer unterschiedlichen Flussgeschwindigkeit des Nährmediums im Randbereich und aufgrund der dort entstehenden Verwirbelungen. Aus diesem Grund ergeben sich für die Verfügbarkeit der Nährstoffe große zonale Unterschiede, die für ein homogenes Wachstum der Zellkulturen ungünstig und daher unerwünscht sind.

Die US 2013/0068310 betrifft eine mehrschichtig aufgebaute Perfusionsvorrichtung mit einer Multiwellplatte. Dabei sind in einer Verteilungsschicht und in einer Gradientenschicht Mikrokanäle angeordnet, die zur Erzeugung eines Konzentrationsgradienten an Nährmedium oberhalb der Zellkultur dienen. Abgegeben wird das Nährmedium über eine poröse Membran. Für die Mikrokanäle der Gradienten- und der Verteilungsschicht sind verschiedene geometrische Anordnungen vorgesehen, wobei die Mikrokanäle über ein Netzwerk an Verbindungskanälen verbunden sind. Dadurch entsteht ein feldähnlicher Konzentrationsgradient an Nährmedium, wobei es insbesondere bei den Umlenkpunkten zu hoher Strömungskompression des Nährmediums kommt und nur in Teilbereichen eine konstante Flussdichte vorliegt. Auch mit dieser Perfusionsvorrichtung können somit aufgrund ungünstiger Nährstoffversorgung keine homogenen Kulturbedingungen für die Zellkulturen zur Verfügung gestellt werden. Für Versuchsreihen mit nachvollziehbaren Ergebnissen ist dies äußerst unerwünscht.

Nachteilig an den im Stand der Technik bekannten Perfusionsvorrichtungen ist vor allem ihre Komplexität. Beispiele derartiger komplexer Perfusionsvorrichtungen sind in der WO 82/03227, WO 02/24861, WO 2015/027998, US 6,670,170, DE 4305405, DE 19742163 und in der DE 10118905 gezeigt. Dies hat zur Folge, dass Einwegsysteme für Perfusionskulturen kaum erhältlich sind, und wenn, dann nur mit derart kleinen Wachstumsoberflächen für die Zellkulturen, dass nur spezielle Einzeluntersuchungen an Zellen sinnvoll und möglich sind. Bei Mehrwegsystemen wiederum verlangen der Betrieb und hier vor allem der sterile Zusammenbau aufgrund der komplexen Vorrichtungen viel Geschick, was üblicherweise zu einem kleinen Probendurchsatz führt.

Die WO 2007/124481 und WO 2011/011350 betreffen Mikrofluidvorrichtungen, wobei über Mikrokanäle Nähr- oder Testsubstanzen zu einzelnen Zellen geführt werden. Zum Teil wird der Fluss der Substanzen ausschließlich durch die in den geschlossen ausgebildeten Mikrokanälen wirkenden Kapillarkräfte bewirkt. Dabei sind die Untersuchungsobjekte entweder in die Wandung des Mikrokanals eingearbeitet oder in eigens dafür angeordneten Halterungen. Da mit diesen Vorrichtungen nur an einzelnen Objekten Untersuchungen durchgeführt werden können, sind die Mikrofluidvorrichtungen geschlossen aufgebaut und weisen eine integrierte Perfusionsvorrichtung auf, die nicht ausgewechselt werden kann. Insbesondere können mit diesen Vorrichtungen nur dynamische aber keine statischen Zellkulturen untersucht werden. Zudem werden durch die Ausgestaltung der Mikrokanäle die Untersuchungsobjekte aus der Mikrofluidvorrichtung zum Teil herausgespült, wodurch ebenfalls homogene Kulturbedingungen für Zellkulturen verunmöglicht werden.

Zusammengefasst ist das Perfusionskulturverfahren mit den im Stand der Technik bekannten Perfusionsvorrichtungen technisch viel aufwendiger und somit teurer zu betreiben als das herkömmliche Batch-Verfahren, wobei vor allem der sterile Zusammenbau und die zeitlich aufwendigere Handhabung der bestehenden Vorrichtungen zu einer geringeren Akzeptanz dieser an sich physiologisch sinnvolleren Alternative zu klassischen in vitro-Modellen geführt hat.

Die Aufgabe der Erfindung liegt somit darin, eine Perfusionsvorrichtung nach Anspruch 1 zur Verfügung zu stellen, mit der die Nachteile der bekannten Vorrichtungen für das Perfusionskulturverfahren vermieden werden. Darüber hinaus soll eine Möglichkeit geschaffen werden, bestehende Zellkulturbehältnisse für statische Kulturen in einfacher Weise in eine Vorrichtung umzuwandeln, mit der statische Kulturen in Perfusionskulturen umgewandelt und mit dem Perfusionskulturverfahren kultiviert werden können. Dabei sollen den Zellkulturen homogene Kulturbedingungen zur Verfügung gestellt werden, wobei die Perfusionsvorrichtung in das Zellkulturbehältnis einsetzbar ist, ohne dass Zellen der Zellkultur zerstört werden.

Mit der erfindungsgemäßen Perfusionsvorrichtung können statische Zellkulturen (sogenannte "batch cultures", "staple culture dishes" oder "staple culture plates") in kürzester Zeit zu Perfusionskulturen (sogenannte "perfusion cultures") umgewandelt bzw. aufgerüstet werden. Auch eine Rückumwandlung in eine statische Zellkultur ist ganz einfach möglich. Zu diesem Zweck weist die erfindungsgemäße Perfusionsvorrichtung einen Perfusionsbereich auf, der über eine Zuleitung mit zumindest einer Einlassöffnung und über eine Ableitung mit zumindest einer Auslassöffnung verbunden ist. Weist die Perfusionsvorrichtung mehrere Einlassöffnungen und/oder mehrere Auslassöffnungen auf, können mehrere Zuleitungen und Ableitungen vorgesehen sein, die zur Verbindung der Einlassöffnungen und/oder der Auslassöffnungen mit dem Perfusionsbereich dienen.

Die zumindest eine Einlassöffnung dient zur Zuführung von Nährmedium, für die zu untersuchenden Zellkulturen, über die zumindest eine Auslassöffnung wird nicht verbrauchtes Nährmedium sowie gegebenenfalls Ausscheidungsstoffe der Zellkulturen abgeführt.

Die Perfusionsvorrichtung weist einen Perfusionsbereich auf, über den Nährmedium an die zu untersuchenden Zellkulturen abgegeben wird, wobei die Zellkulturen in einem Zellkulturbehältnis angeordnet sind, in die der Perfusionsbereich einsetzbar ist. Als Zellkulturbehältnis im Sinne der Erfindung sind dabei herkömmliche, im Stand der Technik bekannte schalenförmige Behältnisse, wie zum Beispiel Petrischalen und zumindest zum Teil plattenförmige Behältnisse, wie zum Beispiel Multiwellplatten sowie andere Behältnisse zu verstehen, die zur Kultivierung von Mikroorganismen und zur Zellkultur verwendet werden, insbesondere Zellkulturbehältnisse mit abnehmbarem Deckel.

Dabei können die Zellen, wie für statische Zellkulturen allgemein üblich, zuerst im Zellkulturbehältnis ausgesät und mit Nährmedium überschichtet werden. Sobald die Zellen am Schalen- oder Plattenboden des Zellkulturbehältnisses angeheftet sind, kann die statische Kultur in eine Perfusionskultur umgewandelt werden, in dem der Perfusionsbereich der erfindungsgemäßen Perfusionsvorrichtung in das Zellkulturbehältnis eingesetzt wird.

Die Einlassöffnung kann mit einem Nährmedienreservoir verbunden werden. Das Nährmedium wird über die Zuleitung zum Perfusionsbereich zugeführt, über den es den Zellkulturen im Zellkulturbehältnis zur Verfügung gestellt wird. Im Falle mehrerer Einlassöffnungen kann Nährmedium über mehrere Zuleitungen dem Perfusionsbereich zugeführt werden. Enden die Zuleitungen an verschiedenen Stellen im Perfusionsbereich, kann - je nach Verteilung der Zuleitungen - eine besonders homogene Abgabe des Nährmediums erzielt werden. Mit mehreren Einlassöffnungen ist es auch möglich, über eine Einlassöffnung Nährmedium zuzuführen, während über eine andere Einlassöffnung mit Medikamenten oder anderen Testsubstanzen versetzte Nährmedien zugeführt werden. Dadurch sind besonders einfach Versuche durchführbar.

Der Perfusionsbereich weist eine geometrische Struktur auf, über welche das Nährmedium den Zellkulturen zur Verfügung gestellt wird. Im eingesetzten Zustand des Perfusionsbereichs ist die Struktur den Zellkulturen zugewandt. Die Struktur bildet einen Kanal aus, der die Zuleitung und die Ableitung verbindet und vom Nährmedium durchströmbar ist. Die geometrische Struktur kann dabei so ausgestaltet sein, dass der gebildete Kanal große Teile des Perfusionsbereichs abdeckt. Damit ist es einerseits möglich, flächenmäßig große Bereiche zur Verfügung zu stellen, die vom Nährmedium durchströmt werden. Somit stehen auch den Zellkulturen im Zellkulturbehältnis flächenmäßig große Bereiche mit Nährmedium zur Verfügung. Andererseits bewirkt der Kanal eine Strömung des Nährmediums mit gewisser Strömungsgeschwindigkeit, sodass die Nachteile statischer Zellkulturverfahren nicht eintreten, dafür aber die Vorteile von Perfusionskulturverfahren.

Der Kanal kann als Rille ausgebildet sein, wobei - je nach geometrischer Struktur - variable Rillentiefe und Rillenbreite aber auch zumindest teilweise oder zur Gänze konstante Rillentiefe und/oder Rillenbreite möglich sind. Indem die geometrische Struktur im eingesetzten Zustand vom Boden des Zellkulturbehältnisses beabstandet ist, kann die Perfusionsvorrichtung eingesetzt werden, ohne die Zellkulturen zu stören oder gar zu zerstören. Dadurch können statische Zellkulturen ohne Beeinträchtigung der Zellen in Perfusionskulturen umgewandelt werden. Der Abstand des äußersten Bereiches der geometrischen Struktur zum Boden des Zellkulturbehältnisses im eingesetzten Zustand des Perfusionsbereichs entspricht dabei dem Abstand des Kanals bzw. der geometrischen Struktur zum Boden des Zellkulturbehältnisses.

Durch die Erfindung ist es möglich die für herkömmliche Zellkulturverfahren verwendeten Petrischalen, aber auch andere Zellkulturbehältnisse wie zum Beispiel Multiwellplatten für Perfusionsverfahren zu verwenden, indem statt dem herkömmlich verwendeten Deckel eine erfindungsgemäße Perfusionsvorrichtung verwendet wird, deren Perfusionsbereich in das Zellkulturbehältnis eingesetzt wird. Die klassischen Formate von Zellkulturbehältnissen können somit beibehalten werden. Durch Abnahme der Perfusionsvorrichtung ist das System von einer Perfusionskultur wieder in eine herkömmliche statische Kultur umwandelbar. Die erfindungsgemäße Perfusionsvorrichtung ist einfachst handhabbar und für ein damit betriebenes Perfusionskulturverfahren besteht kein zeitlicher Nachteil gegenüber des batch-Verfahrens.

Weitere vorteilhafte Ausführungen der Erfindung sind in den abhängigen Ansprüchen definiert.

In einer vorteilhaften Ausführungsform ist die Perfusionsvorrichtung als Deckel für das Zellkulturbehältnis ausgebildet. In diesem Fall kann der normale Deckel, beispielsweise der normale Petrischalendeckel oder die normale Abdeckung der Multiwellplatte entfernt und durch die erfindungsgemäße Perfusionsvorrichtung ersetzt werden. Die gesamte Perfusionsvorrichtung kann dabei in den einfach zu tauschenden Deckel integriert sein. Die Umwandlung einer statischen Zellkultur in eine Perfusionskultur ist somit einfach durch den Tausch des Deckels des Zellkulturbehältnisses möglich. Bevorzugt ist weiter vorgesehen, dass die Perfusionsvorrichtung über ein Halteelement verfügt, mit dem die Perfusionsvorrichtung vom Zellkulturbehältnis abnehmbar ist. Der Tausch des Deckels des Zellkulturbehältnisses ist somit mit einem einzigen Handgriff möglich.

Bevorzugt ist vorgesehen, dass der Kanal und/oder die geometrische Struktur des Perfusionsbereiches im eingesetzten Zustand im Wesentlichen parallel zum Boden des Zellkulturbehältnisses angeordnet ist bzw. sind. Dadurch kann erreicht werden, dass das durch den Kanal durchströmende Nährmedium im Wesentlichen konstanten Abstand zu den Zellkulturen aufweist und somit für die Zellkulturen homogene Umgebungsbedingungen zur Verfügung stehen. In einer Ausführungsform ist dabei vorgesehen, dass die geometrische Struktur im eingesetzten Zustand einen Abstand kleiner als 250 µm zum Boden des Zellkulturbehältnisses aufweist, wobei bevorzugt ein Abstand zwischen 20 µm und 100 µm vorgesehen ist.

In einer weiteren Ausführungsform weist der Perfusionsbereich eine Dichtung zum dichten Abschließen des Zellkulturbehältnisses auf. Beispielsweise ist die Dichtung am Rand des Perfusionsbereiches angeordnet und dichtet im eingesetzten Zustand das Zellkulturbehältnis ab, beispielsweise an der Mantelfläche einer Petrischale. Dadurch können mit einfachen Mitteln die überdruckdichten Verhältnisse wie in einer herkömmlichen komplizierten Perfusionskammer erzielt werden.

In einer Ausführungsform weist die Perfusionsvorrichtung eine Deckplatte auf, an der ein Halteelement zum Abnehmen angeordnet sein kann. Der Perfusionsbereich kann von der Deckplatte beabstandet angeordnet sein. Ist der Perfusionsbereich selbst plattenförmig, kann vorgesehen sein, dass der Perfusionsbereich und die Deckplatte parallel angeordnet sind. Durch eine Beabstandung kann der Perfusionsbereich auch bei Zellkulturbehältnissen verwendet werden, bei denen die Wachstumsoberfläche für die Zellkulturen einen größeren Abstand zum oberen Rand des Behältnisses aufweist. Darüber hinaus ist bei derartigen Perfusionsbereichen ein dichtes Abschließen mit dem Zellkulturbehältnis besonders einfach realisierbar.

Der Perfusionsbereich selbst kann zumindest teilweise oder auch zur Gänze kreisförmig oder zylindrisch ausgebildet sein. Derartige Perfusionsbereiche werden in kreisrunde Petrischalen mit zylinderförmigen Mantelflächen eingesetzt oder beispielsweise in Multiwellplatten mit zylinderförmigen Kammern für die Zellkulturen. Es ist allerdings bei Zellkulturbehältnissen mit anderen Formen, z. B. quaderförmige Behältnisse, auch möglich, dass der Perfusionsbereich eine an das Zellkulturbehältnis angepasste Form aufweist und beispielsweise rechteckig ist.

Bevorzugt ist vorgesehen, dass der durch die geometrische Struktur gebildete Kanal durchgängig ausgebildet ist und, beispielsweise als durchgängige Rille, die Zuleitung mit der Ableitung verbindet. Dabei kann vorgesehen sein, dass das im Perfusionsbereich angeordnete Ende der Zuleitung und/oder das im Perfusionsbereich angeordnete Ende der Ableitung direkt in den Kanal einmündet. Bei einem durchgängigen Kanal ist ein kompletter Umlauf des Nährmediums in einfacherer Weise realisierbar.

Der Kanal ist zumindest teilweise spiralförmig, wobei in einer Ausführungsform der Kanal zur Gänze spiralförmig ausgebildet ist. Dadurch können große Bereiche der Perfusionsvorrichtung abgedeckt werden, wobei eine gleichmäßige Nährstoffversorgung und somit homogene Kulturbedingungen realisiert werden.Die Zuleitung kann in ein Ende des spiralförmigen Teils einmünden, während die Ableitung am anderen Ende des spiralförmigen Teils einmündet. Insbesondere bei kreisförmigen Perfusionsbereichen ist durch einen spiralförmigen Kanal ein großer Teil der Oberfläche des Perfusionsbereiches abdeckbar. Entspricht die Wachstumsoberfläche der Zellkulturen im Zellkulturbehältnis im Wesentlichen der Fläche des Perfusionsbereichs, wird dadurch ein großer Bereich der Wachstumsoberfläche durch das durch den Kanal durchströmende Nährmedium abgedeckt. Es kann vorgesehen sein, dass der Kanal zur Gänze spiralförmig ausgebildet ist. Möglich ist aber auch, dass nur ein gewisser Teil des Kanals spiralförmig ausgebildet ist.

In einer bevorzugten Ausführungsform ist die geometrische Struktur als Steg ausgebildet, der auf dem Perfusionsbereich angeordnet ist. Der Kanal kann in diesem Fall zwischen dem Steg verlaufen bzw. als Raumbereich, der sich zwischen dem Steg ergibt. Der Abstand des äußersten Bereiches des Steges zum Boden des Zellkulturbehältnisses im eingesetzten Zustand des Perfusionsbereichs entspricht dabei dem Abstand zum Boden des Zellkulturbehältnisses.

In einer bevorzugten Ausführungsform weist der Steg gekrümmte Seitenflanken auf. Die Seitenflanken des Stegs können dabei über einen flachen Verbindungsbereich verbunden sein oder aber zu einer mehr oder weniger scharfen Kante zusammenlaufen. Auch ein gekrümmter Übergang ohne scharfe Kanten ist möglich. Durch die gekrümmten Seitenflanken können Sauerstoffgradienten, die insbesondere bei statischen Zellkulturverfahren ein Problem darstellen, vermieden werden.

Der Kanal kann als Rille zwischen der als Steg ausgeformten geometrischen Struktur ausgebildet sein. Der Steg bildet die Wandung des Kanals. Bei gekrümmten Seitenflanken kann sich dabei ein im Querschnitt gekrümmter Kanal, beispielsweise ein halbkreisförmiger Kanal, ergeben. Aber auch andere Geometrien, wie etwa teilweise elliptisch oder anderweitig ovale Kanäle, sind möglich. Insbesondere kann eine sich verändernde Krümmung vorgesehen sein. An seinem äußersten Bereich kann es vorgesehen sein, dass der Steg ebenfalls gekrümmt, z. B. nach außen gewölbt, ausgebildet ist, sodass der Übergang zwischen zwei Rillen keine scharfen Kanten aufweist, um Gradientenbildung zu vermeiden.

In einer bevorzugten Ausführungsform ist der Kanal nach unten hin offen ausgebildet. Das Nährmedium kann dadurch direkt und ungehindert zu den Zellkulturen gelangen, wodurch wiederum eine Gradientenbildung vermieden werden kann.

In einer Ausführungsform der Erfindung beträgt die Querschnittsfläche des Kanals zwischen 2,5 mm² und 20 mm², vorzugsweise zwischen 8 mm² und 16 mm². Die Höhe der geometrischen Struktur bzw. die Höhe des Steges beträgt zwischen 0,5 mm und 5 mm, vorzugsweise zwischen 2 mm und 2,5 mm. Unter der Höhe der geometrischen Struktur bzw. der Höhe des Steges soll dabei die Tiefe des Kanals verstanden werden. Das ist jenes Maß, das im eingesetzten Zustand der Perfusionsvorrichtung senkrecht zum Boden des Zellkulturbehältnisses angeordnet ist und vom äußersten Punkt des Stegs bis zum tiefsten Punkt der Rille gemessen wird.

Der durch die geometrische Struktur gebildete Kanal ermöglicht eine Strömungsgeschwindigkeit für das Nährmedium. Der Kanal kann dabei derart ausgebildet sein, um Medienflüsse von bis zu 10 ml/min zu ermöglichen, wobei Medienflüsse zwischen 0,2 ml/h und 1 ml/h bevorzugt werden, um zellstörende Scherkräfte zu vermeiden. Der Kanal ermöglicht eine homogene Perfusion über die gesamte Wachstumsoberfläche ohne Gradientenzonierung. Dies ergibt sich insbesondere bei einer gekrümmten Kanalform.

Die geometrische Struktur kann derart ausgebildet sein, dass das Nährmedium zwischen den Rillen diffundiert. Dadurch kann ein Druckaufbau im System vermieden werden, während die Mikrozirkulation und die Umwälzung des Nährmediums gefördert wird.

Zum selben Zweck kann der Steg und/oder der Kanal Vorrichtungen aufweisen, die zur Strömungsumlenkung für das durchströmende Nährmedium dienen. Dabei kann es sich um kleine Barrieren oder Schikanen handeln. Die Vorrichtungen zur Strömungsumlenkung verbessern ebenfalls die Mikrozirkulation und die Umwälzung des Nährmediums im Kanal. Darüber hinaus wird die Überströmung von unverbrauchtem Nährmedium optimiert, sodass sich eine verbesserte Gas- und Nährstoffverfügbarkeit für die kultivierten Zellen ergibt.

Die Einlassöffnung und/oder die Auslassöffnung können mit einer Pumpe verbunden werden, mit der die Strömung des Nährmediums erwirkt wird und frisches Nährmedium zugeführt bzw. verbrauchtes Nährmedium abgeführt wird. Möglich sind hier beispielsweise Perfusionspumpen, Peristaltikpumpen, Spritzenpumpen und dergleichen mehr.

In einer weiteren Ausführungsform ist vorgesehen, dass die Perfusionsvorrichtung eine erste und eine zweite Einlassöffnung zur Zuführung von Nährmedium sowie eine erste und eine zweiter Auslassöffnung zur Abführung von noch nicht verbrauchtem Nährmedium aufweist. Dabei kann beispielsweise in der ersten Einlassöffnung Nährmedium zugeführt werden, während in der zweiten Einlassöffnung Medikamente oder andere Testsubstanzen zugeführt werden. Es ist aber auch möglich, dass sowohl mit der ersten, als auch mit der zweiten Einlassöffnung ausschließlich Nährmedium oder bereits mit Medikamenten bzw. Testsubstanzen versetztes Nährmedium zugeführt wird. Dabei ist der Perfusionsbereich mit der ersten Einlassöffnung über eine erste Zuleitung und mit der zweiten Einlassöffnung über eine zweite Zuleitung verbunden. Denkbar ist aber auch, dass die erste und die zweite Einlassöffnung in dieselbe Zuleitung münden. Der Perfusionsbereich ist mit der ersten Auslassöffnung über eine erste Ableitung und mit der zweiten Auslassöffnung über eine zweite Ableitung verbunden. Es ist aber auch denkbar, dass sowohl die erste als auch die zweite Auslassöffnung in eine Ableitung münden. Darüber hinaus ist grundsätzlich auch möglich, mehr als zwei Einlassöffnungen und mehr als zwei Auslassöffnungen vorzusehen. Ferner muss die Anzahl der Einlassöffnungen mit der Anzahl der Auslassöffnungen nicht übereinstimmen.

Indem eine Mehrzahl von Einlassöffnungen vorgesehen ist, ist es möglich, die Zuführung des Nährmediums noch mehr zu homogenisieren, beispielsweise indem die Zuleitungen auf dem Perfusionsbereich entsprechend verteilt sind. Darüber hinaus können durch eine Mehrzahl von Einlassöffnungen zonale Untersuchungen durchgeführt werden, indem über die einzelnen Einlassöffnungen unterschiedliche Nährstoffe oder unterschiedliche Medikamente bzw. andere Testsubstanzen zugeführt werden, die dann über die entsprechenden Zuleitungen zum Perfusionsbereich geleitet und den Zellkulturen dort zur Verfügung gestellt werden. In den entsprechenden Zonen ergeben sich dann unterschiedliche Reaktionen der Zellkultur.

Bei zumindest zwei Einlass- und zwei Auslassöffnungen ist bevorzugt vorgesehen, dass die geometrische Struktur die erste Zuleitung mit der ersten Ableitung in Form eines ersten Kanals und die zweite Zuleitung mit der zweiten Ableitung in Form eines zweiten Kanals verbindet. Dabei ist bevorzugt vorgesehen, dass der erste Kanal und der zweite Kanal vom Nährmedium gegenläufig durchströmbar sind. Durch die Gegenströmung werden die Umwälzung des Nährmediums und die Mikrozirkulation weiter verbessert.

Um großflächige Bereiche des Perfusionsbereichs abdecken zu können, kann vorgesehen sein, den ersten und den zweiten zumindest teilweise spiralförmig ausgebildeten Kanal zumindest zur Gänze spiralförmig auszubilden, wobei gegenläufige Spiralen bevorzugt sind.

Die Erfindung betrifft weiters ein Set umfassend eine wie oben beschriebene Perfusionsvorrichtung sowie ein Zellkulturbehältnis, insbesondere eine Petrischale.

Im Falle eines Zellkulturbehältnisses mit mehreren Kammern zur Kultivierung von Zellen, insbesondere in Form einer Multiwellplatte, kann vorgesehen sein, dass die Perfusionsvorrichtung mehrere Perfusionsbereiche aufweist, wobei jeweils ein Perfusionsbereich in einer Kammer einsetzbar ist.

Weitere Einzelheiten und Vorteile der vorliegenden Erfindung werden anhand der Figurenbeschreibung unter Bezugnahme auf die Zeichnungen im Folgenden näher erläutert. Darin zeigt:
- Fig. 1: eine schematische Darstellung einer Vorrichtung für eine statische Zellkultur nach dem Stand der Technik,
- Fig. 2a - 2c: eine perspektivische Ansicht, eine Seitenansicht und eine Draufsicht von unten einer erfindungsgemäßen Perfusionsvorrichtung,
- Fig. 3a und 3b: zwei Querschnittsdarstellungen der erfindungsgemäßen Perfusionsvorrichtu ng,
- Fig. 4a - 4g: eine perspektivische Ansicht, zwei Seitenansichten, eine Draufsicht von unten, eine Draufsicht von oben und zwei Querschnittsdarstellungen einer weiteren Ausführungsform einer erfindungsgemäßen Perfusionsvorrichtung,
- Fig. 5a - 5g: eine perspektivische Ansicht, zwei Seitenansichten, eine Draufsicht von unten, eine Draufsicht von oben und zwei Querschnittsdarstellungen einer weiteren Ausführungsform einer erfindungsgemäßen Perfusionsvorrichtung,
- Fig. 6a - 6h: eine perspektivische Ansicht, zwei Seitenansichten, eine Draufsicht von unten, eine Draufsicht von oben und drei Querschnittsdarstellungen einer weiteren Ausführungsform einer erfindungsgemäßen Perfusionsvorrichtung,
- Fig. 7a - 7c: schematische Darstellungen von Vorrichtungen zur Strömungsumlenkung für das Nährmedium und
- Fig. 8a und 8b: schematische Darstellungen zweier erfindungsgemäßer Sets.

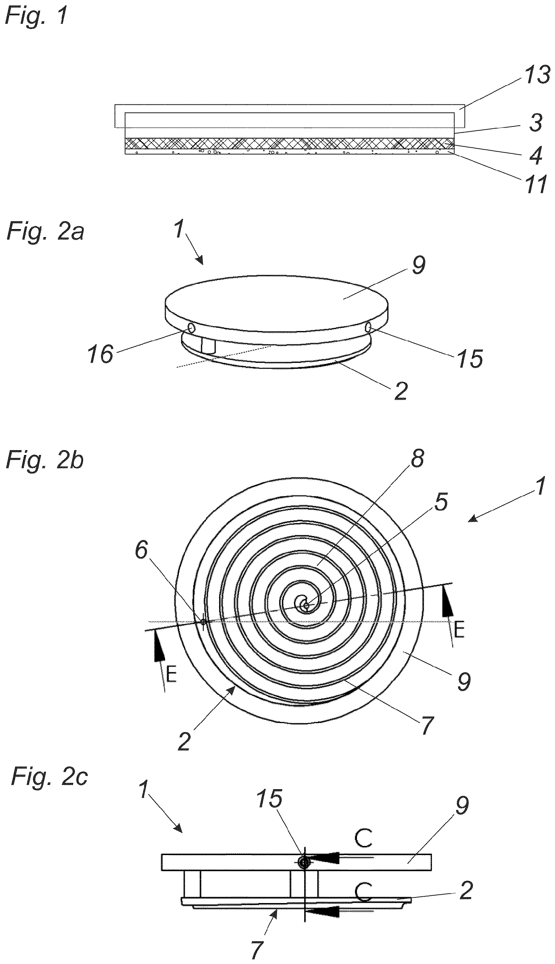

Figur 1 zeigt eine Vorrichtung für eine statische Zellkultur, wobei Zellen 11 am Boden eines als Petrischale ausgeführten Zellkulturbehältnisses 3 angeordnet sind und mit Nährmedium 4 überschichtet sind. Das Zellkulturbehältnis 3 ist mit einem Deckel 13 abgeschlossen.

Der Deckel 13 kann durch eine erfindungsgemäße Perfusionsvorrichtung 1 ersetzt werden, wodurch das bestehende Zellkulturbehältnis 3 samt darin bereits kultivierter Zellen 11 für das Perfusionskulturverfahren herangezogen werden kann.

In der perspektivischen Ansicht der Figur 2a ist eine erfindungsgemäße Perfusionsvorrichtung 1 dargestellt, die eine Deckplatte 9 und einen davon beabstandeten Perfusionsbereich 2 aufweist. Der Perfusionsbereich 2 ist ebenso wie die Deckplatte 9 kreisrund ausgebildet und kann beispielsweise in ein Zellkulturbehältnis 3 in Form einer kreisrunden Petrischale eingesetzt werden. Gezeigt ist weiters die Einlassöffnung 15, über die Nährmedium 4 zugeführt werden kann und die Auslassöffnung 16, über die nicht verbrauchtes Nährmedium bzw. die Ausscheidungsstoffe der Zellen 11 abgeführt werden kann.

Figur 2b zeigt eine Draufsicht von unten auf die Perfusionsvorrichtung 1. In der Mitte des kreisrunden Perfusionsbereichs 2 ist die Zuleitung 5 dargestellt, über die der Perfusionsbereich 2 mit der Einlassöffnung 15 verbunden ist. Die Zuleitung 5 mündet in den spiralförmigen Kanal 8, der durch die geometrische Struktur 7 in Form eines spiralförmigen Stegs gebildet wird. Durch die Spiralform des Kanals 8 werden große Bereiche des Perfusionsbereichs 2 abgedeckt und können vom Nährmedium 4 durchströmt werden. Der Perfusionsbereich 2 mit der daran angeordneten geometrischen Struktur 7 stellt eine Perfusionsschnecke dar, wobei der Kanal 8 durch die Rillen der Schnecke gebildet wird.

In dieser Ausführungsform sind die Zuleitung 5 und die Ableitung 6 zumindest teilweise als Rohrverbindungen ausgebildet, wobei die Rohre die Beabstandung des Perfusionsbereiches 2 von der Deckplatte 9 ermöglichen.

Figur 2c zeigt eine Seitenansicht der erfindungsgemäßen Perfusionsvorrichtung 1, an der der Abstand zwischen der Deckplatte 9 und dem ebenfalls plattenförmigen Perfusionsbereich 2 sowie die am Perfusionsbereich 2 angeordnete geometrische Struktur 7 gut erkennbar sind. Dargestellt ist weiters die Einlassöffnung 15.

Die erfindungsgemäße Perfusionsvorrichtung 1 kann als steriles Einwegsystem konzipiert sein und beispielsweise im Spritzgussverfahren hergestellt werden. Als Material kommen bevorzugt Kunststoffe, wie z. B. Polystyrol, PE-Kunststoffe, PP-Kunststoffe, PET-Kunststoffe, PTFE-Kunststoffe und dergleichen mehr in Frage.

Nachdem der Perfusionsbereich 2 in das Zellkulturbehältnis 3 eingesetzt ist, können an sich für Perfusionsverfahren im Stand der Technik bekannte Gerätschaften angeschlossen werden, um ein Perfusionskulturverfahren zu betreiben. Die Einlassöffnung 15 oder die Auslassöffnung 16 kann mit einer Pumpe, bevorzugt eine Mehrkanal-Perfusionspumpe, verbunden werden, wofür Schläuche, bevorzugt gasimpermeable Schläuche, wie z. B. Neopren, und Anschlussadapter, bevorzugt verschraubbare Luer-Einwegverbinder, verwendet werden können. Ein Nährmedien-Reservoir, z. B. ein Wasserbad oder ein Brutschrank, dient zur Versorgung der Zellkultur mit Nährmedium 4, das gegebenenfalls zusätzlich begast wird.

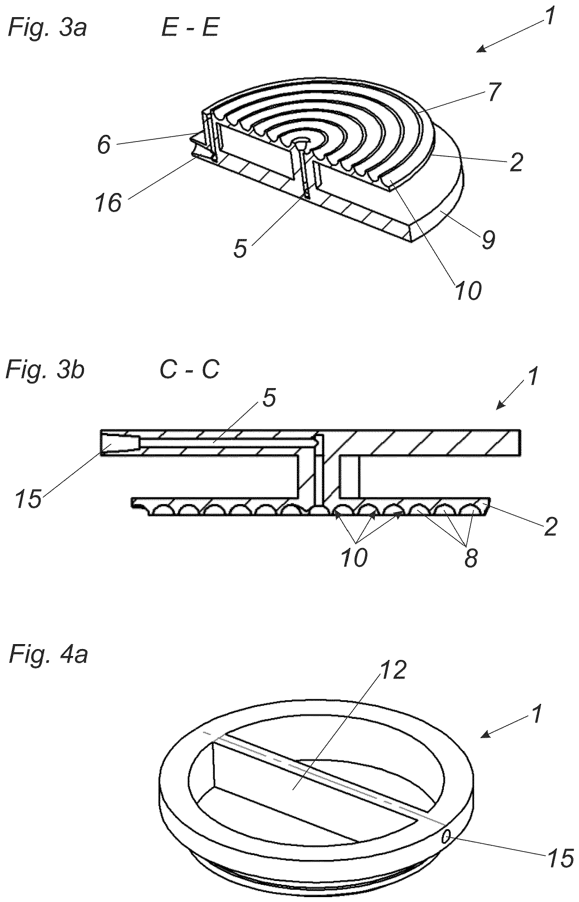

Figur 3a zeigt eine Querschnittsdarstellung der Perfusionsvorrichtung 1 entlang der Schnittlinie EE gemäß Figur 2b. Erkennbar ist, dass die als Steg ausgebildete geometrische Struktur 7 gekrümmte Seitenflanken 10 aufweist, sodass der Kanal 8 einen halbkreisförmigen Querschnitt aufweist. Erkennbar ist weiters die Zuleitung 5, die die Einlassöffnung 15 mit dem Perfusionsbereich 2 verbindet und dabei in den spiralförmigen Kanal 8 mündet, sowie die Ableitung 6, die am äußeren Rand des spiralförmigen Kanals 8 angeordnet ist und welche die Auslassöffnung 16 mit dem Perfusionsbereich 2 verbindet.

Figur 3b zeigt eine Querschnittsdarstellung entlang der Schnittlinie CC gemäß Figur 2c. Wiederum ist der halbkreisförmige Querschnitt des Kanals 8 erkennbar, der durch gekrümmte Seitenflanken 10 der als Steg ausgebildeten geometrische Struktur 7 gebildet wird. Erkennbar ist die als Rohverbindung ausgebildete Zuleitung 5, die die Einlassöffnung 15 mit dem Perfusionsbereich 2 verbindet.

Alternativ kann die Einlassöffnung 15 und die Auslassöffnung 16 auch vertauscht werden, sodass das frische Nährmedium 4 am äußeren Rand des spiralförmigen Kanals 8 zugeführt wird.

In den Figuren 4a bis 4f ist eine weitere Ausführungsform der erfindungsgemäßen Perfusionsvorrichtung dargestellt, die sich im Wesentlichen dadurch von der Perfusionsvorrichtung 1 gemäß der Figuren 2a bis 2d und 3a und 3b unterscheidet, dass die Deckplatte 9 ein Halteelement 12 aufweist, mit dem die Perfusionsvorrichtung 1 samt Perfusionsbereich 2 mit einem Handgriff in das Zellkulturbehältnis 3 eingesetzt werden kann.

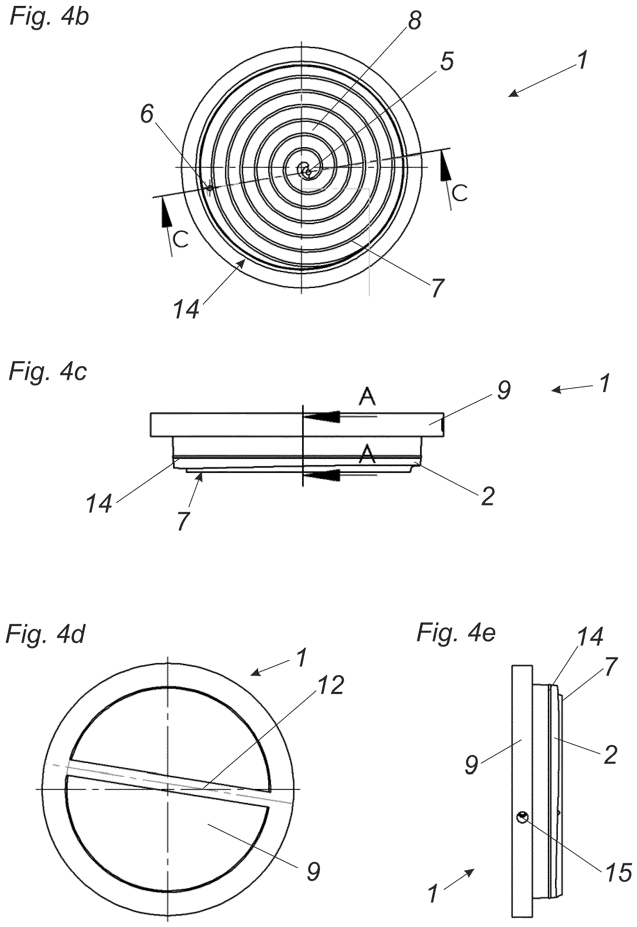

In den Seitenansichten gemäß Figur 4c und Figur 4e ist eine Dichtung 14 dargestellt, die um den Rand des Perfusionsbereiches 2 angeordnet ist und zum dichten Abschließen eines kreisrunden Zellkulturbehältnisses 3, beispielsweise einer Petrischale, dient. Figur 4b zeigt eine Draufsicht dieser Ausführungsform der Perfusionsvorrichtung 1 von unten, die im Wesentlichen der Darstellung gemäß Figur 2b entspricht. Figur 4d zeigt eine Draufsicht von oben, bei der das Halteelement 12 erkennbar ist.

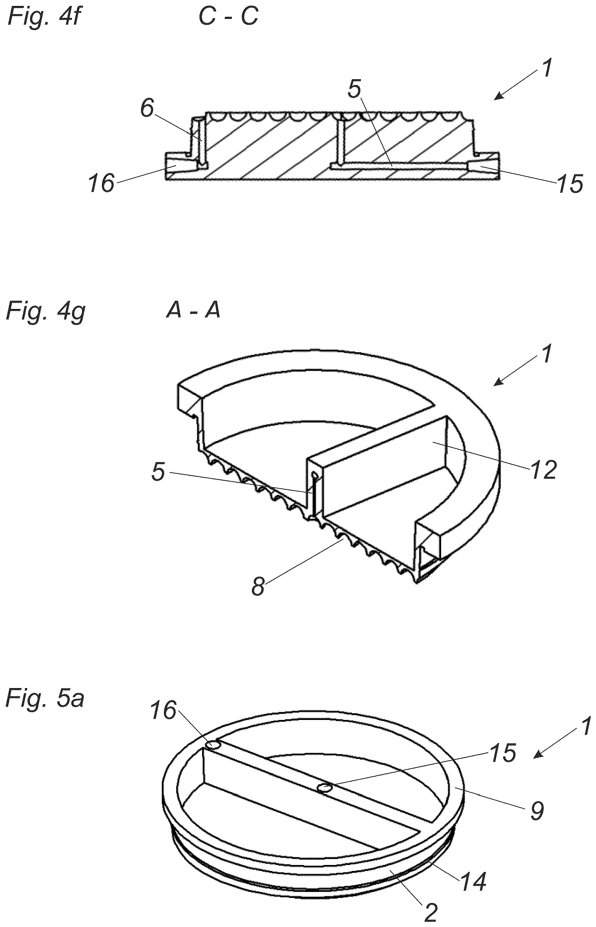

Figur 4f zeigt eine Querschnittdarstellung entlang der Schnittlinie CC gemäß Figur 4b. Erkennbar ist die in dieser Ausführungsform kanalförmig ausgebildete Zuleitung 5 und die kanalförmig ausgebildete Ableitung 6, mit der die Einlassöffnung 15 und die Auslassöffnung 16 mit dem Perfusionsbereich 2 verbunden sind.

Figur 4g zeigt eine Querschnittsdarstellung entlang der Schnittlinie AA gemäß Figur 4c, in der wiederum die Zuleitung 5 und der halbkreisförmige Querschnitt des Kanals 8 erkennbar sind, der durch die gekrümmten Seitenflanken 10 der als Steg ausgebildeten geometrischen Struktur 7 gebildet wird.

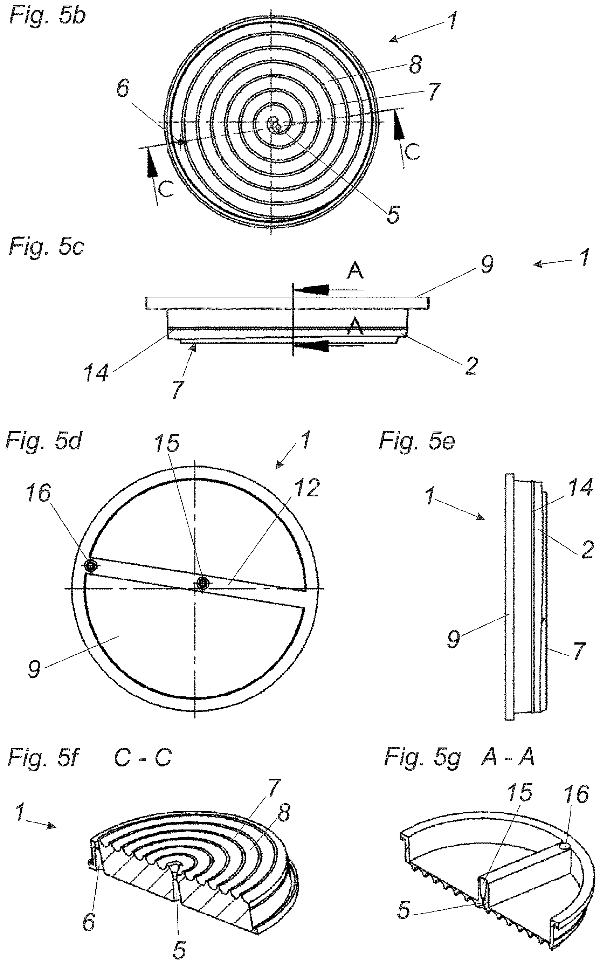

Die in den Figuren 5a bis 5g dargestellte Ausführungsform der erfindungsgemäßen Perfusionsvorrichtung 1 unterscheidet sich von der in den Figuren 4a bis 4g dargestellten Ausführungsform im Wesentlichen nur dadurch, dass sowohl die Einlassöffnung 15 als auch die Auslassöffnung 16 auf der Oberseite der Deckplatte 9 und nicht auf der Seitenfläche angeordnet sind.

Wie anhand der in der Figur 5f gezeigten Querschnittsdarstellung entlang der Schnittlinie CC gemäß Figur 5b dargestellt ist, ist diese Anordnung der Einlassöffnung 15 und der Auslassöffnung 16 mit dem Vorteil verbunden, dass die Zuleitung 5 und die Ableitung 6 weniger kompliziert und mit kürzeren, in dieser Ausführungsform kanalförmigen Abschnitten realisiert werden können. Dies ist auch in der Querschnittsdarstellung der Figur 5g gezeigt, die einen Querschnitt entlang der Schnittlinie AA gemäß Figur 5c zeigt. In dieser Darstellung ist die Zuleitung 5 erkennbar, die unmittelbar in die Einlassöffnung 15 übergeht. Sowohl die Einlassöffnung 15 als auch die Auslassöffnung 16 sind direkt oberhalb der Einmündung der Zuleitung 5 und der Ableitung 6 in den Perfusionsbereich 2 angeordnet.

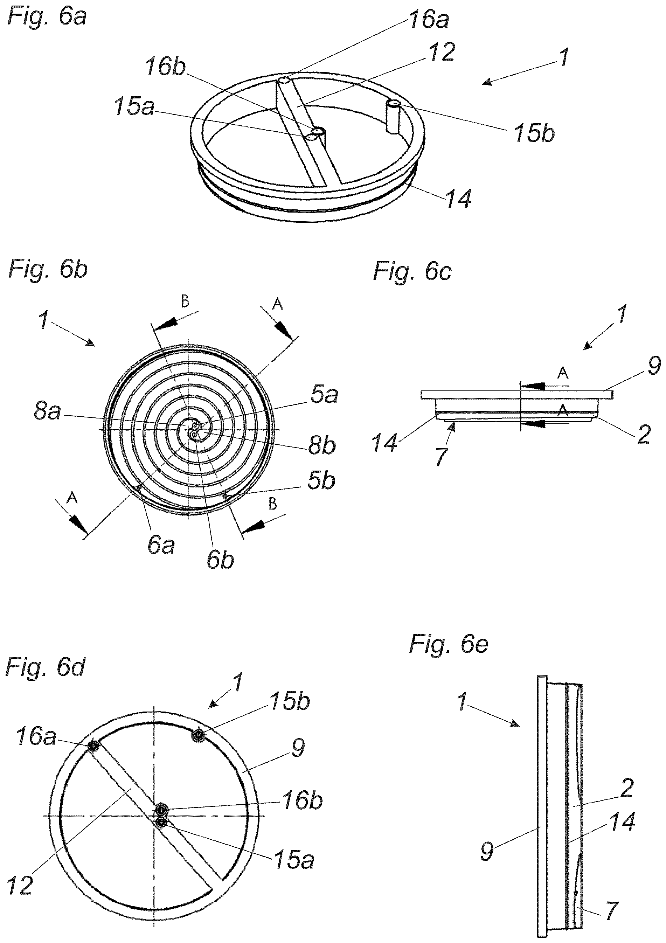

In den Figuren 6a bis 6h ist eine weitere Ausführungsform der erfindungsgemäßen Perfusionsvorrichtung 1 gezeigt. Wiederum weist die Perfusionsvorrichtung 1 eine kreisrunde Deckplatte 9 mit einem Halteelement 12 zum einfachen Einsetzen des ebenfalls kreisrunden Perfusionsbereiches 2 in ein Zellkulturbehältnis 3 auf. Der Perfusionsbereich 2 ist von einer Dichtung 14 umrandet, die zum dichten Abschließen des Zellkulturbehältnisses 3 dient.

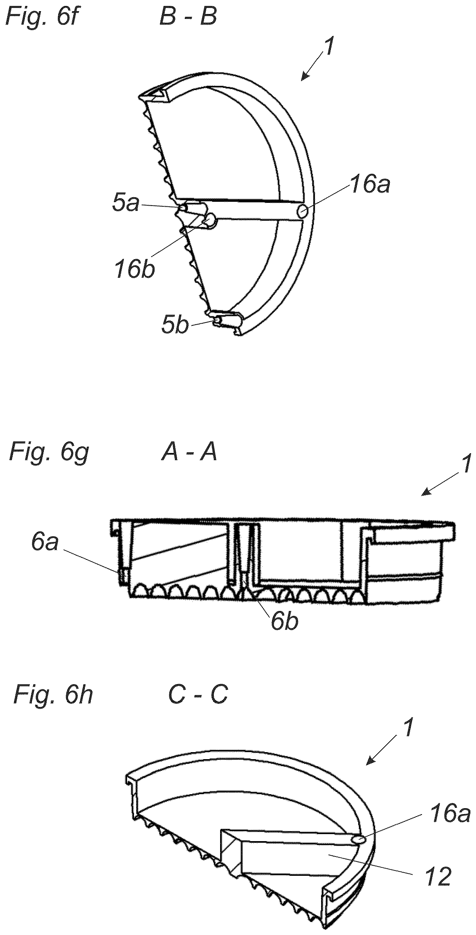

In diesem Fall weist die Perfusionsvorrichtung 1 eine erste Einlassöffnung 15a auf, die mittig in der Deckplatte 9 angeordnet ist. Eine zweite Einlassöffnung 15b ist am Rand der Deckplatte 9 angeordnet. Die erste Einlassöffnung 15a und die zweite Einlassöffnung 15b sind über eine erste Zuleitung 5a und eine zweite Zuleitung 5b mit dem Perfusionsbereich 2 verbunden. Die erste Zuleitung 5a und die zweite Zuleitung 5b sind besonders gut in der Querschnittsdarstellung gemäß Figur 6f gezeigt, die einen Querschnitt entlang der Schnittlinie BB gemäß Figur 6b zeigt. Da die erste Einlassöffnung 15a und die zweite Einlassöffnung 15b direkt oberhalb auf der Oberseite der Deckplatte 9 angeordnet sind, können die erste Zuleitung 5a und die zweite Zuleitung 5b kurz gehalten sein.

Eine erste Auslassöffnung 16a ist am Rand der Deckplatte angeordnet, während eine zweite Auslassöffnung 16b mittig in der Nähe der ersten Einlassöffnung 15a angeordnet ist. Die erste Auslassöffnung 16a und die zweite Auslassöffnung 16b sind über eine erste Ableitung 6a und eine zweite Ableitung 6b mit dem Perfusionsbereich 2 verbunden. Dies ist gut in der Querschnittsdarstellung der Figur 6g erkennbar, die einen Querschnitt entlang der Schnittlinie AA gemäß Figur 6b zeigt. Wiederum können die erste Ableitung 6a und die zweite Ableitung 6b kurz gehalten sein, da die erste Auslassöffnung 16a und die zweite Auslassöffnung 16b direkt oberhalb an der Oberseite der Deckplatte 9 angeordnet sind.

Wie anhand der Draufsicht von unten gemäß Figur 6b erkennbar ist, mündet die erste Zuleitung 5a in einen ersten Kanal 8a, in den auch die erste Ableitung 6a mündet. Die zweite Zuleitung 5b mündet wie die zweite Ableitung 6b in einen zweiten Kanal 8b. Sowohl der erste Kanal 8a als auch der zweite Kanal 8b sind spiralförmig ausgebildet. Der erste Kanal 8a und der zweite Kanal 8b werden durch die Zwischenräume eines spiralförmigen Steges gebildet, der die geometrische Struktur 7 des Perfusionsbereiches 2 darstellt.

Generell tritt das Nährmedium 4 und/oder eine Testsubstanz bzw. Medikamente bei der Zuleitung 5 in den Kanal 8 ein und durchströmt diesen in Richtung der Ableitung 6, wo das nicht verbrauchte Nährmedium 4 gegebenenfalls gemeinsam mit Ausscheidungsprodukten der Zellen 11 austritt.

Im vorliegenden Fall tritt das Nährmedium 4 bei der ersten Zuleitung 5a in den ersten Kanal 8a ein und durchströmt diesen in Richtung der ersten Ableitung 6a, wo es aus dem ersten Kanal 8a austritt. Das Nährmedium 4 und/oder eine Testsubstanz bzw. Medikamente tritt bei der zweiten Zuleitung 5b in den zweiten Kanal 8b ein und durchströmt diesen in Richtung der zweiten Ableitung 6b, wo das nicht verbrauchte Nährmedium gegebenenfalls mit Ausscheidungsprodukten der Zellen 11 aus dem Perfusionsbereich 2 austritt. Da in dieser Ausführungsform die erste Zuleitung 5a mittig angeordnet ist, während die zweite Zuleitung 5b am äußeren Rand angeordnet ist, werden der erste Kanal 8a und der zweite Kanal 8b gegenläufig vom Nährmedium 4 durchströmt.

Werden beispielsweise entlang des Kanals 8a und 8b insgesamt 20 % des zugeführten Nährmediums 4 verbraucht, d. h. 80 % des zugeführten frischen Nährmediums 4 treten bei der Auslassöffnung wieder aus, so ist es aufgrund der gegenläufigen Durchströmung möglich, dass im Mittel rund 90 % frisches Nährmedium 4 den Zellen 11 zur Verfügung gestellt wird. Es herrschen somit äußerst homogene Umgebungsbedingungen für die Zellen 11, was sich für die Untersuchungen des Verhaltens der Zellen 11 beispielsweise unter Zuführung gewisser Medikamente oder Testsubstanzen als äußerst vorteilhaft herausgestellt hat. Die Gegenläufigkeit des ersten spiralförmigen Kanals 8a und des zweiten spiralförmigen Kanals 8b ist besonders gut in der Figur 6b erkennbar.

Figur 6h zeigt eine Querschnittsdarstellung entlang der Schnittlinie CC gemäß Figur 6c.

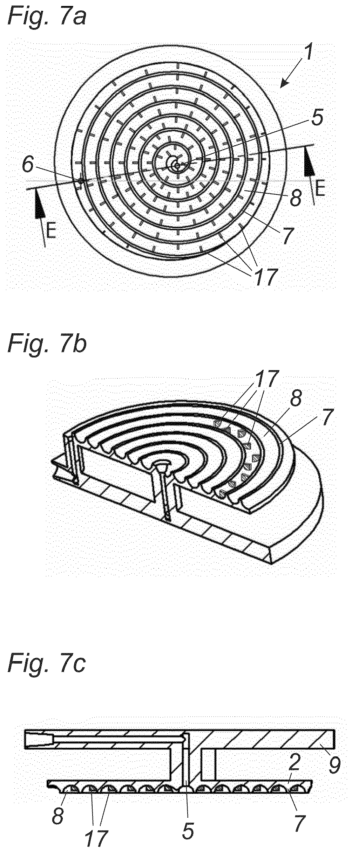

Figur 7a zeigt in einer Draufsicht eine weitere Ausführungsform einer erfindungsgemäßen Perfusionsvorrichtung 1 mit einer als spiralförmigen Steg ausgebildeten geometrischen Struktur 7, deren Zwischenräume einen ebenfalls spiralförmigen Kanal 8 ausbilden, der vom Nährmedium 4 durchströmbar ist, wobei das Nährmedium 4 bei einer in den Kanal 8 einmündenden Zuleitung 5 einströmt und bei einer ebenfalls in den Kanal 8 einmündenden Ableitung 6 ausströmt. Auf beiden Seiten des Stegs sind Vorrichtungen 17 zur Strömungsumlenkung in Form von Schikanen angeordnet, die zu einer Mikrozirkulation und besseren Umwälzung des durchströmenden Nährmediums 4 führen.

Figur 7b zeigt eine schematische Querschnittsdarstellung, bei der die in den Seitenflanken 10 des Stegs angeordneten Vorrichtungen 17 zur Strömungsumlenkung angeordnet sind, mit der die Mikrozirkulation des durchströmenden Nährmediums 4 erhöht wird.

Figur 7c zeigt eine Querschnittsdarstellung entlang der Schnittlinie EE gemäß Figur 7a, bei der ebenfalls die Vorrichtungen 17 zur Strömungsumlenkung erkennbar sind, die als Schikanen an den Seitenflanken 10 der als Steg ausgebildeten geometrischen Struktur 7 angeordnet sind.

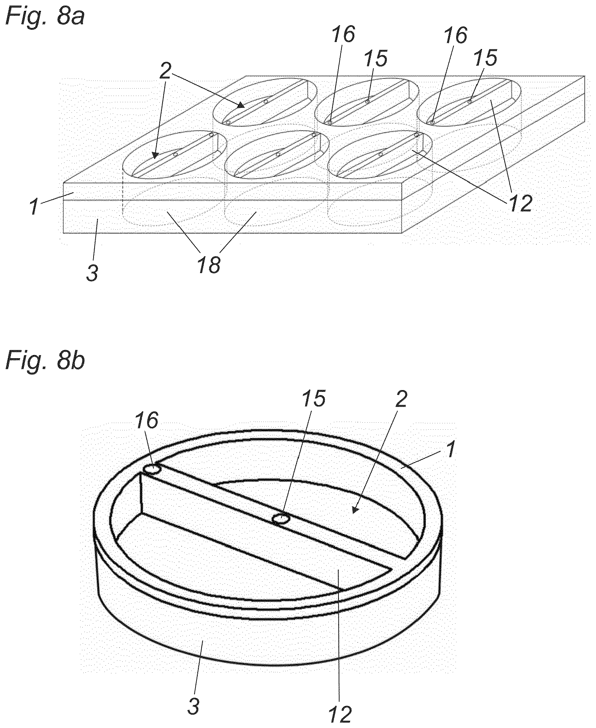

Figur 8a zeigt eine perspektivische Darstellung eines erfindungsgemäßen Sets umfassend ein als Multiwellplatte ausgebildetes Zellkulturbehältnis 3, welches über eine Vielzahl von strichliert dargestellten kammerförmigen Vertiefungen 18 verfügt, auf deren Boden Zellkulturen 11 angeordnet werden. Die Perfusionsvorrichtung 1 kann als Deckel für die Multiwellplatte dienen. Die Perfusionsvorrichtung 1 weist eine Mehrzahl von Perfusionsbereichen 2 auf, wobei jeder Perfusionsbereich 2 in eine der kammerförmigen Vertiefungen 18 eingesetzt wird. Die Perfusionsvorrichtung 1 weist darüber hinaus eine Mehrzahl von Halteelementen 12 auf, die jeweils einen Perfusionsbereich 2 zugeordnet sind. Darüber hinaus ist jedem Perfusionsbereich 2 eine Einlassöffnung 15 und eine Auslassöffnung 16 zugeordnet. Die Einlassöffnung 15 und die Auslassöffnung 16 sind über eine Zuleitung 5 und eine Ableitung 6 mit dem jeweiligen Perfusionsbereich 2 verbunden, sodass jede einzelne kammerförmige Vertiefung 18 mit Nährmedium 4 versorgt werden kann. Da jeder kammerförmigen Vertiefung 18 je eine Einlassöffnung 15 und eine Auslassöffnung 16 zugeordnet sind, können die einzelnen kammerförmigen Vertiefungen 18 mit unterschiedlichem Nährmedien versorgt werden, sodass gleichzeitig mehrere unterschiedliche Versuche durchgeführt werden können.

Figur 8b zeigt eine schematische Darstellung eines erfindungsgemäßen Sets, bestehend aus einem als Petrischale ausgebildeten Zellkulturbehältnis 3 und einer Perfusionsvorrichtung 1, deren Perfusionsbereich 2 in die Petrischale eingesetzt ist.

## Patentansprüche

1. Perfusionsvorrichtung (1) für ein Zellkulturbehältnis (3), insbesondere eine Petrischale, mit zumindest einer Einlassöffnung (15, 15A, 15B) zur Zuführung von Nährmedium und zumindest einer Auslassöffnung (16, 16A, 16B) zur Abführung von nicht verbrauchtem Nährmedium (4), wobei wenigstens ein, insbesondere plattenförmiger, Perfusionsbereich (2) zur Abgabe von Nährmedium an Zellkulturen vorgesehen ist, der mit der Einlassöffnung (15,15A,15B) über eine Zuleitung (5) verbunden ist und mit der Auslassöffnung (16, 16a, 16B) über eine Ableitung (6) verbunden ist, wobei der Perfusionsbereich (2) in das Zellkulturbehältnis (3) einsetzbar ist, **dadurch gekennzeichnet, dass** der Perfusionsbereich (2) eine geometrische Struktur (7) aufweist, welche die Zuleitung (5) und die Ableitung (6) in Form eines zumindest teilweise, vorzugsweise zur Gänze, spiralförmig ausgebildeten Kanals (8) verbindet, welcher vom Nährmedium (4) durchströmbar ist, wobei die geometrische Struktur (7) im eingesetzten Zustand vom Boden des Zellkulturbehältnisses (3) beabstandet ist.

2. Perfusionsvorrichtung nach Anspruch 1, wobei die Perfusionsvorrichtung (1) als Deckel (13) für das Zellkulturbehältnis (3) ausgebildet ist und vorzugsweise eine Dichtung (14) zum dichten Abschließen des Zellkulturbehältnisses (3) aufweist und/oder wobei der Perfusionsbereich (2) zumindest teilweise kreisförmig oder zylindrisch ausgebildet ist.

3. Perfusionsvorrichtung nach Anspruch 1 oder 2, wobei der Kanal (8) des Perfusionsbereichs (2) und/oder die geometrische Struktur (7) im eingesetzten Zustand im Wesentlichen parallel zum Boden des Zellkulturbehältnisses (3) angeordnet ist bzw. sind.

4. Perfusionsvorrichtung nach Anspruche 3, wobei die geometrische Struktur (7) im eingesetzten Zustand einen Abstand kleiner als 250 µm, vorzugsweise zwischen 20 µm und 100 µm, zum Boden des Zellkulturbehältnisses (3) aufweist.

5. Perfusionsvorrichtung nach einem der Ansprüche 1 bis 4, wobei die Perfusionsvorrichtung (1) eine Deckplatte (9) aufweist und der Perfusionsbereich (2) von der Deckplatte (9) beabstandet angeordnet ist.

6. Perfusionsvorrichtung nach einem der Ansprüche 1 bis 5, wobei der Kanal (8) durchgängig ausgebildet ist und/oder wobei der Kanal (8) nach unten offen ausgebildet ist.

7. Perfusionsvorrichtung nach einem der Ansprüche 1 bis 6, wobei die geometrische Struktur (7) als Steg ausgebildet ist, der auf dem Perfusionsbereich (2) angeordnet ist, wobei der Kanal (8) zwischen dem Steg verläuft und vorzugsweise gekrümmte Seitenflanken (10) aufweist.

8. Perfusionsvorrichtung nach Anspruch 7, wobei der Kanal (8) eine Querschnittsfläche zwischen 2,5 mm² und 20 mm², vorzugsweise zwischen
8 mm² und 16 mm², aufweist und/oder wobei der Steg eine Höhe zwischen 0,5 mm und 5 mm, vorzugsweise zwischen 2 mm und 2,5 mm, aufweist.

9. Perfusionsvorrichtung nach Anspruch 7 oder 8, wobei der Steg und/oder der Kanal (8) Vorrichtungen (17) zur Strömungsumlenkung für das durchströmende Nährmedium (4) aufweist.

10. Perfusionsvorrichtung nach einem der Ansprüche 1 bis 9, wobei eine erste Einlassöffnung (15a) und eine zweite Einlassöffnung (15b) zur Zuführung von Nährmedium (4) und eine erste Auslassöffnung (16a) und eine zweite Auslassöffnung (16b) zur Abführung von nicht verbrauchtem Nährmedium vorgesehen sind, und wobei der Perfusionsbereich (2)
- mit der ersten Einlassöffnung (15a) über eine erste Zuleitung (5a) verbunden ist,
- mit der zweiten Einlassöffnung (15b) über eine zweite Zuleitung (5b) verbunden ist
- mit der ersten Auslassöffnung (16a) über eine erste Ableitung (6a) verbunden ist, und
- mit der zweiten Auslassöffnung (16b) über eine zweite Ableitung (6b) verbunden ist.

11. Perfusionsvorrichtung nach Anspruch 10, wobei die geometrische Struktur (7) die erste Zuleitung (5a) mit der ersten Ableitung (6a) in Form eines ersten, zumindest teilweise, vorzugsweise zur Gänze, spiralförmig ausgebildeten, Kanals (8a) und die zweite Zuleitung (5b) mit der zweiten Ableitung (6b) in Form eines zweiten, zumindest teilweise, vorzugsweise zur Gänze, spiralförmig ausgebildeten Kanals (8b) verbindet, und wobei der erste Kanal (8a) und der zweite Kanal (8b) vom Nährmedium (4), vorzugsweise gegenläufig, durchströmbar sind.

12. Perfusionsvorrichtung nach Anspruch 11, wobei der erste Kanal (8a) und der zweite Kanal (8b) als gegenläufige Spiralen ausgebildet sind.

13. Perfusionsvorrichtung nach einem der Ansprüche 1 bis 12, wobei ein Halteelement (12) vorgesehen ist, mit dem die Perfusionsvorrichtung (1) von dem Zellkulturbehältnis (3) abnehmbar ist.

14. Set umfassend ein Zellkulturbehältnis, insbesondere eine Petrischale, und eine Perfusionsvorrichtung (1) nach einem der Ansprüche 1 bis 13, wobei der Per,fusionsbereich (2) in das Zellkulturbehältnis (3) einsetzbar ist.

15. Set nach Anspruch 14, wobei das Zellkulturbehältnis (3) mehrere Kammern zur Kultivierung von Zellen (11) aufweist, insbesondere in Form einer Multiwellplatte, wobei die Perfusionsvorrichtung (1) mehrere Perfusionsbereiche (2) aufweist, und wobei jeweils ein Perfusionsbereich (2) in eine Kammer einsetzbar ist.

## Claims

1. A perfusion device (1) for a cell culture container (3), in particular a petri dish, comprising at least one inlet opening (15, 15a, 15b) for the supply of nutrient medium and at least one outlet opening (16, 16a, 16b) for the discharge of unused nutrient medium (4), wherein there is provided at least one, preferably plate-shaped, perfusion region (2) for the delivery of nutrient medium to cell cultures, which is connected to the inlet opening (15, 15a, 15b) by way of a supply line (5) and to the outlet opening (16, 16a, 16b) by way of a discharge line (6), wherein the perfusion region (2) can be inserted into the cell culture container (3), **characterised in that** the perfusion region (2) comprises a geometrical structure (7) which connects the supply line (5) and the discharge line (6) in the form of an at least partially, preferably entirely, spiral-shaped passage (8) through which the nutrient medium (4) can flow, wherein the geometrical structure (7) is spaced from the bottom of the cell culture (3) container in the inserted state.

2. A perfusion device according to claim 1 wherein the perfusion device (1) is in the form of a cover (13) for the cell culture container (3), preferably having a seal (14) for sealingly closing off the cell culture container (3), and/or wherein the perfusion region (2) is at least partially of a circular or cylindrical configuration.

3. A perfusion device according to claim 1 or 2 wherein the passage (8) of the perfusion region (2) and/or the geometrical structure (7) in the inserted state is or are arranged substantially parallel to the bottom of the cell culture container (3).

4. A perfusion device according to claim 3 wherein the geometrical structure (7) in the inserted state is at a spacing of less than 250 µm, preferably between 20 µm and 100 µm, relative to the bottom of the cell culture container (3).

5. A perfusion device according to one of the claims 1 to 4 wherein the perfusion device (1) has a cover plate (9) and the perfusion region (2) is arranged spaced from the cover plate (9).

6. A perfusion device according to one of the claims 1 to 5 wherein the passage (8) is of a continuous configuration and/or downwardly open.

7. A perfusion device according to one of the claims 1 to 6 wherein the geometrical structure (7) is in the form of a web, which is arranged on the perfusion region (2), wherein the passage (8) extends between the web, preferably having curved side flanks (10).

8. A perfusion device according to claim 7 wherein the passage (8) has a cross-sectional area of between 2.5 mm² and 20 mm², preferably between 8 mm² and 16 mm² and/or wherein the web is of a height of between 0.5 mm and 5 mm, preferably between 2 mm and 2.5 mm.

9. A perfusion device according to claim 7 or 8 wherein the web and/or the passage has devices (17) for flow deflection for the through-flow of nutrient medium (4).

10. A perfusion device according to one of the claims 1 to 9 wherein there are provided a first inlet opening (15a) and a second inlet opening (15b) for the supply of nutrient medium (4) and a first outlet opening (16a) and a second outlet opening (16b) for the discharge of unused nutrient medium, and wherein the perfusion region (2)
- is connected to the first inlet opening (15a) by way of a first supply line (5a),
- is connected to the second inlet opening (15b) by way of a second supply line (5b),
- is connected to the first outlet opening (16a) by way of a first discharge line (6a), and
- is connected to the second outlet opening (16b) by way of a second discharge line (6b).

11. A perfusion device according to claim 10 wherein the geometrical structure (7) connects the first supply line (5a) to the first discharge line (6a) in the form of a first, at least partially, preferably entirely, spiral-shaped passage (8a) and the second supply line (5b) to the second discharge line (6b) in the form of a second, at least partially, preferably entirely, spiral-shaped passage (8b), and wherein the nutrient medium (4) can flow through the first passage (8a) and the second passage (8b), preferably in opposite relationship.

12. A perfusion device according to claim 11 wherein the first passage (8a) and/or the second passage (8b) are in the form of oppositely directed spirals.

13. A perfusion device according to one of the claims 1 to 12 wherein there is provided a holding element (12) with which the perfusion device (1) is removable from the cell culture container (3).

14. A set including a cell culture container, in particular a petri dish, and a perfusion device (1) according to one of the claims 1 to 13, wherein the perfusion region (2) can be inserted into the cell culture container (3).

15. A set according to claim 14 wherein the cell culture container (3) has a plurality of chambers (11) for the cultivation of cells, in particular in the form of a multi-well plate, wherein the perfusion device (1) has a plurality of perfusion regions (2) and wherein a respective perfusion region (2) can be inserted into a chamber.

## Revendications

1. Dispositif de perfusion (1) pour un récipient de culture cellulaire (3), en particulier une boîte de Pétri, avec au moins une ouverture d'entrée (15, 15A, 15B) pour l'amenée de milieu nutritif et au moins une ouverture de sortie (16, 16A, 16B) pour l'évacuation de milieu nutritif (4) non consommé, dans lequel au moins une zone de perfusion (2), en particulier en forme de plaque, est prévue pour la distribution de milieu nutritif aux cultures cellulaires, qui est reliée à l'ouverture d'entrée (15, 15A, 15B) par le biais d'une conduite d'amenée (5) et est reliée à l'ouverture de sortie (16, 16A, 16B) par le biais d'une conduite d'évacuation (6), dans lequel la zone de perfusion (2) peut être insérée dans le récipient de culture cellulaire (3), **caractérisé en ce que** la zone de perfusion (2) présente une structure géométrique (7) qui relie la conduite d'amenée (5) et la conduite d'évacuation (6) sous la forme d'un canal (8) réalisé au moins partiellement, de préférence entièrement, en spirale qui peut être traversé par le milieu nutritif (4), dans lequel la structure géométrique (7) est espacée dans l'état inséré du fond du récipient de culture cellulaire (3).

2. Dispositif de perfusion selon la revendication 1, dans lequel le dispositif de perfusion (1) est réalisé comme couvercle (13) pour le récipient de culture cellulaire (3) et présente de préférence une garniture (14) pour la fermeture étanche du récipient de culture cellulaire (3) et/ou dans lequel la zone de perfusion (2) est réalisée au moins partiellement de manière circulaire ou cylindrique.

3. Dispositif de perfusion selon la revendication 1 ou 2, dans lequel le canal (8) de la zone de perfusion (2) et/ou la structure géométrique (7) est ou sont agencé(s) dans l'état inséré sensiblement parallèlement au fond du récipient de culture cellulaire (3).

4. Dispositif de perfusion selon la revendication 3, dans lequel la structure géométrique (7) présente dans l'état inséré une distance inférieure à 250 µm, de préférence entre 20 µm et 100 µm par rapport au fond du récipient de culture cellulaire (3).

5. Dispositif de perfusion selon l'une des revendications 1 à 4, dans lequel le dispositif de perfusion (1) présente une plaque de recouvrement (9) et la zone de perfusion (2) est agencée de manière espacée de la plaque de recouvrement (9).

6. Dispositif de perfusion selon l'une des revendications 1 à 5, dans lequel le canal (8) est réalisé de manière continue et/ou dans lequel le canal (8) est réalisé ouvert vers le bas.

7. Dispositif de perfusion selon l'une des revendications 1 à 6, dans lequel la structure géométrique (7) est réalisée comme nervure qui est agencée sur la zone de perfusion (2), dans lequel le canal (8) s'étend entre la nervure et présente des flancs latéraux (10) de préférence courbés.

8. Dispositif de perfusion selon la revendication 7, dans lequel le canal (8) présente une aire de section entre 2,5 mm² et 20 mm², de préférence entre 8 mm² et 16 mm², et/ou dans lequel la nervure présente une hauteur entre 0,5 mm et 5 mm, de préférence entre 2 mm et 2,5 mm.

9. Dispositif de perfusion selon la revendication 7 ou 8, dans lequel la nervure et/ou le canal (8) présente des dispositifs (17) pour la déviation d'écoulement pour le milieu nutritif (4) circulant.

10. Dispositif de perfusion selon l'une des revendications 1 à 9, dans lequel une première ouverture d'entrée (15a) et une deuxième ouverture d'entrée (15b) pour l'amenée de milieu nutritif (4) et une première ouverture de sortie (16a) et une deuxième ouverture de sortie (16b) pour l'évacuation de milieu nutritif non consommé sont prévues, et dans lequel la zone de perfusion (2)
- est reliée à la première ouverture d'entrée (15a) par le biais d'une première conduite d'amenée (5a),
- est reliée à la deuxième ouverture d'entrée (15b) par le biais d'une deuxième conduite d'amenée (5b),
- est reliée à la première ouverture de sortie (16a) par le biais d'une première conduite d'évacuation (6a), et
- est reliée à la deuxième ouverture de sortie (16b) par le biais d'une deuxième conduite d'évacuation (6b).

11. Dispositif de perfusion selon la revendication 10, dans lequel la structure géométrique (7) relie la première conduite d'amenée (5a) à la première conduite d'évacuation (6a) sous la forme d'un premier canal (8a) réalisé, au moins partiellement, de préférence entièrement, en spirale et la deuxième conduite d'amenée (5b) à la deuxième conduite d'évacuation (6b) sous la forme d'un deuxième canal (8b) réalisé, au moins partiellement, de préférence entièrement, en spirale, et dans lequel le premier canal (8a) et le deuxième canal (8b) peuvent être traversés par le milieu nutritif (4), de préférence en sens inverse.

12. Dispositif de perfusion selon la revendication 11, dans lequel le premier canal (8a) et le deuxième canal (8b) sont réalisés comme des spirales en sens inverse.

13. Dispositif de perfusion selon l'une des revendications 1 à 12, dans lequel un élément de retenue (12) est prévu, avec lequel le dispositif de perfusion (1) peut être retiré du récipient de culture cellulaire (3).

14. Ensemble comprenant un récipient de culture cellulaire, en particulier une boîte de Pétri, et un dispositif de perfusion (1) selon l'une des revendications 1 à 13, dans lequel la zone de perfusion (2) peut être insérée dans le récipient de culture cellulaire (3).

15. Ensemble selon la revendication 14, dans lequel le récipient de culture cellulaire (3) présente plusieurs chambres pour la culture de cellules (11), en particulier sous la forme d'une plaque multipuits, dans lequel le dispositif de perfusion (1) présente plusieurs zones de perfusion (2), et dans lequel respectivement une zone de perfusion (2) peut être insérée dans une chambre.
